(19) Europäisches Patentamt
European Patent Office
Office européen des brevets

(11) Veröffentlichungsnummer: **0 079 850**
**A1**

# (12) EUROPÄISCHE PATENTANMELDUNG

(21) Anmeldenummer: **82810419.0**

(22) Anmeldetag: **11.10.82**

(51) Int. Cl.³: **C 07 D 498/10**
C 07 D 498/20, C 08 K 5/35
//(C07D498/10, 263/00, 221/00), (C07D498/20, 263/00, 221/00, 221/00)

(30) Priorität: **15.10.81 CH 6600/81**

(43) Veröffentlichungstag der Anmeldung:
**25.05.83 Patentblatt 83/21**

(84) Benannte Vertragsstaaten:
**CH DE FR GB IT LI**

(71) Anmelder: **CIBA-GEIGY AG**
**Patentabteilung Postfach**
**CH-4002 Basel(CH)**

(72) Erfinder: **Karrer, Friedrich, Dr.**
**Rebbergstrasse 5**
**CH-4800 Zofingen(CH)**

(54) **N-Substituierte Polyalkylpiperidin-4-spirooxazolone.**

(57) Verbindungen der Formeln Ia und Ib

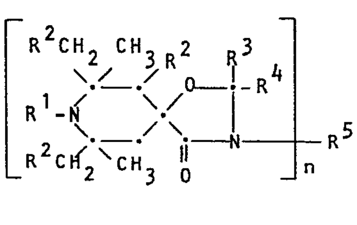

(Ia)

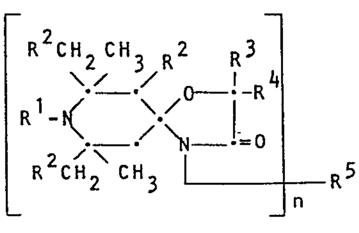

(Ib)

worin n 1 oder 2 ist, $R^1$ ein aliphatischer oder aromatischer Acylrest, Carbamoylrest oder $-CH_2CN$ ist und $R^2$, $R^3$, $R^4$ und $R^5$ die im Anspruch 1 gegebenen Bedeutungen haben, weisen eine viel geringere Basizität auf als entsprechende bekannte Verbindungen, in denen $R^1$ H oder ein Kohlenwasserstoffrest ist, sind aber in ihrer Lichtschutzwirkung den bekannten Verbindungen mindestens ebenbürtig. Für bestimmte Substrate ist das ein wesentlicher Vorteil.

EP 0 079 850 A1

Croydon Printing Company Ltd

CIBA-GEIGY AG
Basel (Schweiz)

3-13598/S /=

## N-Substituierte Polyalkylpiperidin-4-spirooxazolone

Die Erfindung betrifft neue Polyalkylpiperidin-4-spirooxazolone und ihre Verwendung als Stabilisatoren für organische Polymere, insbesondere gegen Einwirkung von Licht, sowie die so stabilisierten Polymeren.

Aus den DE-OS 26 06 026, 28 34 962, 29 33 732 und der EP-A 17 617 sind bereits Polyalkylpiperidin-4-spirooxazolone der Formel A und B

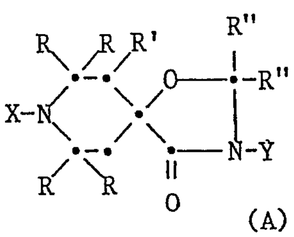
(A)

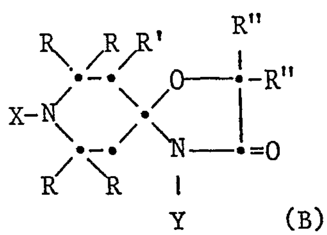
(B)

als Lichtschutzmittel für organische Polymere bekannt, worin R Alkyl, R' Wasserstoff oder Alkyl, R'' einwertige Kohlenwasserstoffreste, X Wasserstoff, Sauerstoff, OH, oder einen gegebenenfalls substituierten Kohlenwasserstoffrest und Y Wasserstoff oder einen Kohlenwasserstoffrest bedeuten. Alle diese Verbindungen besitzen ein basisches Stickstoff-Atom im Piperidinring und sind daher basische Verbindungen, die mit Säuren Salze bilden können.

Für bestimmte Anwendungsgebiete kann die Basizität dieser Verbindungen von Nachteil sein, beispielsweise für säurekatalysierte Lacke oder für elektrostatisch oder elektrophoretisch applizierte Lacke. Es bestand daher das Bedürfnis nach nicht-basischen Polyalkylpiperidin-4-spirooxazolonen für solche speziellen Einsatzgebiete.

Es wurde gefunden, dass sich hierfür solche Verbindungen der Formel A oder B eignen, in denen X eine Acyl-, Carbamoyl- oder Cyanomethylgruppe ist. Gegenstand der Erfindung sind daher Verbindungen der Formel Ia oder Ib,

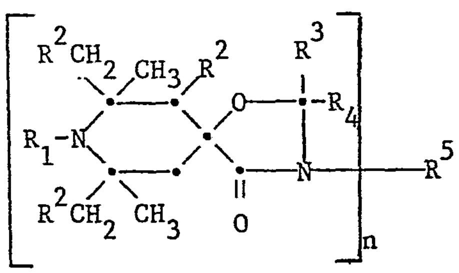

(Ia)

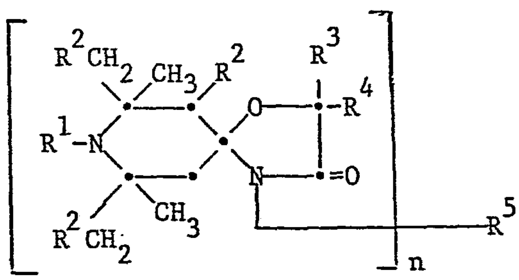

(Ib)

worin n 1 oder 2 ist,

$R^1$ $C_2$-$C_{30}$-Alkanoyl, $C_3$-$C_{20}$-Alkenoyl, $-CH_2CN$, $-CO-N(R^6)(R^7)$ $C_7$-$C_{11}$-Aroyl, $C_8$-$C_{14}$-Aryl-alkanoyl oder $C_8$-$C_{20}$ Alkyl-aroyl bedeutet,

$R^2$ Wasserstoff oder $C_1$-$C_4$-Alkyl ist,

$R^3$ und $R^4$ unabhängig voneinander Wasserstoff, $C_1$-$C_{30}$-Alkyl, unsubstituiertes oder durch Cl oder Alkyl substituiertes $C_6$-$C_{10}$-Aryl, unsubstituiertes oder durch Alkyl substituiertes $C_7$-$C_{11}$-Aralkyl bedeuten oder $R^3$ und $R^4$ zusammen mit dem C-Atom, an das sie gebunden sind, einen Cycloalkan- oder Alkylcycloalkanring mit 5-18 C-Atomen oder eine Gruppe der Formel

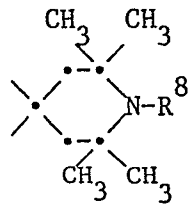

bilden,

$R^5$ wenn n = 1, $C_1$-$C_{30}$-Alkyl, $C_3$-$C_{20}$-Alkenyl, unsubstituiertes oder durch Alkyl substituiertes $C_7$-$C_{11}$-Aralkyl oder $C_5$-$C_{12}$-Cycloalkyl, $C_2$-$C_{18}$-Alkanoyl, $C_3$-$C_{18}$-Alkenoyl, $C_7$-$C_{11}$-Aroyl, $C_8$-$C_{14}$-Aryl-alkanoyl oder $C_8$-$C_{20}$ Alkyl-aroyl bedeutet,

$R^5$ wenn n = 2, $C_2$-$C_{30}$-Alkylen, $C_2$-$C_{30}$-Alkenylen oder $C_8$-$C_{18}$-Arylen-dialkylen bedeutet,

$R^6$ Wasserstoff, $C_1$-$C_{18}$-Alkyl, $C_5$-$C_8$-Cycloalkyl, $C_7$-$C_{15}$-Aralkyl oder $C_6$-$C_{10}$-Aryl bedeutet,

$R^7$ $C_1$-$C_{18}$-Alkyl, $C_5$-$C_8$-Cycloalkyl, $C_7$-$C_{15}$-Aralkyl oder $C_6$-$C_{10}$-Aryl bedeutet, oder

$R^6$ und $R^7$ zusammen mit dem N-Atom einen 5-7-gliedrigen, gegebenenfalls durch weitere Heteroatome unterbrochenen Ring bilden und

$R^8$ Wasserstoff, $C_1$-$C_{12}$-Alkyl, $C_3$-$C_5$-Alkenyl, $C_3$-$C_5$-Alkinyl, unsubstituiertes oder durch Alkyl substituiertes $C_7$-$C_{11}$-Aralkyl, $C_2$-$C_{18}$-Alkanoyl, $C_3$-$C_{18}$-Alkenoyl, $-CH_2CN$ oder $-CO-N(R^6)(R^7)$ bedeutet.

$R^2$, $R^3$, $R^4$, $R^5$, $R^6$, $R^7$ und $R^8$ können unverzweigtes oder verzweigtes Alkyl sein. Beispiele hierfür - im Rahmen der definierten C-Anzahl - sind die Gruppen Methyl, Ethyl, Isopropyl, sec. Butyl, tert. Butyl, Hexyl, Octyl, Dodecyl, Tetradecyl, Heptadecyl, Octadecyl, Eikosyl, Docosyl oder Triakontyl.

$R^5$ und $R^8$ als Alkenyl können - im Rahmen der definierten C-Anzahl - z.B. Allyl, Methallyl, 3,3-Dimethyl-allyl, 1-Buten-4-yl, 2-Hepten-1-yl oder Oleyl sein.

$R^8$ als Alkinyl kann z.B. Propargyl, Butinyl oder Pentinyl sein.

$R^5$, $R^6$ und $R^7$ als Cycloalkyl können z.B. Cyclopentyl, Cyclohexyl oder Cyclooctyl sein. $R^5$ kann auch z.B. Methyl-cyclohexyl, Dimethyl-cyclopentyl oder Cyclododecyl sein.

$R^3$, $R^4$, $R^6$ und $R^7$ als Aryl können Phenyl oder Naphthyl sein, $R^3$ und $R^4$ können auch z.B. Chloraryl oder durch Alkyl substituiertes Aryl sein, insbesondere durch $C_1$-$C_4$ substituiertes Phenyl. Beispiele hierfür sind Chlorphenyl, Chlornaphthyl, Mono- oder Dimethylphenyl, Nonylphenyl oder Dodecylphenyl.

$R^3$, $R^4$, $R^5$, $R^6$, $R^7$ und $R^8$ als Aralkyl können z.B. Phenylethyl, Phenylisopropyl, insbesondere aber Benzyl sein. Beispiele für $R^3$, $R^4$, $R^5$ und $R^8$ als alkyliertes Aralkyl sind Methyl-naphthylmethyl, Octylbenzyl, Nonylbenzyl, Dodecylbenzyl, insbesondere aber durch $C_1$-$C_4$-Alkyl substituiertes Benzyl wie z.B. Methylbenzyl, Isopropylbenzyl oder tert. Butylbenzyl.

Wenn $R^3$ und $R^4$ zusammen mit dem C-Atom, an das sie gebunden sind, einen Cycloalkanring bilden, so kann dieser z.B. ein Cyclohexan, Cyclooctan oder Cyclododecanring sein.

Wenn $R^6$ und $R^7$ zusammen mit dem N-Atom, an das sie gebunden sind, einen 5-7-gliedrigen Ring bilden, so kann dies z.B. ein Pyrrolidin-, Piperidin-, Morpholin- oder Piperazinring sein.

$R^5$ als Alkylen kann unverzweigtes oder verzweigtes Alkylen sein wie z.B. Ethylen, Hexamethylen, Octamethylen, Dodecamethylen, 1,2-Propylen oder 2,2-Dimethylpropylen-1,3. $R^5$ als Alkenylen kann z.B. 2-Butenylen-1,4- oder 2,3-Dimethyl-2-butenylen-1,4 sein. $R^5$ als Arylendialkylen kann z.B. Phenylendiethylen oder Naphthylendimethylen, insbesondere aber m- oder p-Xylylen sein.

$R^1$, $R^5$ und $R^8$ als aliphatische Acylreste können z.B. Acetyl, Propionyl, Butyryl, Hexanoyl, Octanoyl, Dodecanoyl oder Octadecanoyl (Stearoyl) sein. $R^1$ kann darüber hinaus auch z.B. Eikosanoyl, Docosanoyl, Triakontanoyl, Acryloyl, Methacryloyl, Crotonoyl, Hexenoyl oder Octadecenoyl (Oleoyl) sein.

$R^1$ und $R^5$ als aromatische Acylreste können z.B. Benzoyl, Naphthoyl, Phenylacetyl, Phenylpropionyl, Naphthylacetyl, Toluyl, Dimethylbenzoyl, 4-Butylbenzoyl, 4-Octylbenzoyl oder 4-Dodecylbenzoyl sein.

Bevorzugt sind Verbindungen der Formel Ia oder Ib, worin

$R^1$ $C_2$-$C_{12}$-Alkanoyl, $C_3$-$C_5$-Alkenoyl, Phenylacetyl, $-CH_2CN$ oder $-CO-N(R^6)(R^7)$ bedeutet,

$R^2$ Wasserstoff ist,

$R^3$ und $R^4$ unabhängig voneinander Wasserstoff, $C_1$-$C_{12}$-Alkyl, $C_7$-$C_{12}$-Aralkyl oder durch $C_1$-$C_4$-Alkyl substituiertes Phenyl bedeuten oder $R^3$ und $R^4$ zusammen mit dem C-Atom, an das sie gebunden sind, einen Cycloalkan- oder Alkylcycloalkanring mit 5-12 C-Atomen oder eine Gruppe der Formel

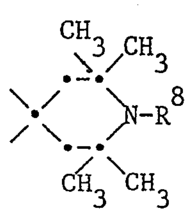

bilden,

$R^5$ wenn n=1, $C_1$-$C_{18}$-Alkyl, $C_3$-$C_6$-Alkenyl, $C_5$-$C_6$-Cycloalkyl, $C_7$-$C_{12}$-Aralkyl, $C_2$-$C_{12}$-Alkanoyl oder $C_3$-$C_{12}$-Alkenoyl, und wenn n = 2, $C_2$-$C_{18}$-Alkylen, $C_4$-$C_{12}$-Alkenylen oder Xylylen bedeutet,

$R^6$ Wasserstoff, $C_1$-$C_{12}$-Alkyl, $C_5$-$C_6$-Cycloalkyl, $C_7$-$C_{12}$-Aralkyl oder Phenyl bedeutet,

$R^7$ $C_1$-$C_{12}$-Alkyl, $C_5$-$C_6$-Cycloalkyl, $C_7$-$C_{12}$-Aralkyl oder $C_6$-$C_{10}$-Aryl bedeutet oder $R^6$ und $R^7$ zusammen mit dem N-Atom einen 5-7-gliedrigen Ring bilden und

$R^8$ Wasserstoff, $C_1$-$C_6$-Alkyl, $C_3$-$C_5$-Alkenyl, Benzyl, $C_2$-$C_{12}$-Alkanoyl, $C_3$-$C_{12}$-Alkenoyl, Propargyl oder Cyanomethyl bedeutet.

Besonders bevorzugt sind Verbindungen der Formel Ia, worin n = 1 ist, $R^1$ $C_2$-$C_{12}$-Alkanoyl, $C_3$-$C_5$-Alkenoyl, $-CO-N(R^6)(R^7)$ oder $-CH_2CN$ bedeutet, $R^2$ Wasserstoff ist, $R^3$ und $R^4$ unabhängig voneinander H, $C_1$-$C_4$-Alkyl, Benzyl oder Phenyl bedeuten oder $R^3$ und $R^4$ zusammen mit dem C-Atom, an das sie gebunden sind, einen $C_5$-$C_{12}$-Cycloalkanring bilden, $R^5$ $C_1$-$C_6$-Alkyl, $C_3$-$C_5$-Alkenyl, Benzyl, $C_2$-$C_{12}$-Alkanoyl oder $C_3$-$C_5$-Alkenoyl bedeutet, $R^6$ H, $C_1$-$C_6$-Alkyl, Cyclohexyl, Benzyl oder Phenyl bedeutet und $R^7$ $C_1$-$C_6$-Alkyl, Cyclohexyl, Benzyl oder Phenyl bedeutet oder $R^6$ und $R^7$ zusammen mit dem N-Atom einen 5-7-gliedrigen Ring bilden.

Beispiele für Verbindungen der Formel Ia mit n = 1 sind:

8-Acetyl-2,2,7,7,9,9-hexamethyl-1-oxa-3,8-diaza-4-oxo-spiro[4.5]decan,

8-Acryloyl-2,2,7,7,9,9-hexamethyl-1-oxa-3,8-diaza-4-oxo-spiro[4.5]-decan,

3-Methacryloyl-2,2,4,4-tetramethyl-7-oxa-3,14-diaza-15-oxo-dispiro-[5.1.5.2]pentadecan,

3-Acryloyl-2,2,4,4-tetramethyl-20-allyl-7-oxa-3,20-diaza-21-oxo-dispiro[5.1.11.2]heneicosan,

8-Acryloyl-2,2,7,7,9,9-hexamethyl-3-benzyl-1-oxa-3,8-diaza-4-oxo-spiro[4,5]decan,

8-Acryloyl-7,7,9,9-tetramethyl-3-octadecyl-2,2-diethyl-1-oxa-3,8-diaza-4-oxo-spiro[4,5]decan,

3-Acetyl-2,2,4,4-tetramethyl-20-allyl-7-oxa-3,20-diaza-21-oxo-dispiro[5.1.11.2]heneicosan,

3-Acryloyl-2,2,4,4-tetramethyl-20-benzyl-7-oxa-3,20-diaza-21-oxo-dispiro[5.1.11.2]heneicosan,

3,20-Diheptadecanoyl-2,2,4,4-tetramethyl-7-oxa-3,20-diaza-21-oxo-dispiro[5.1.11.2]heneicosan,

3-Acetyl-2,2,4,4-tetramethyl-20-benzyl-7-oxa-3,20-diaza-21-oxo-dispiro[5.1.11.2]heneicosan,

3-(N,N-Diäthyl)-carbamoyl-2,2,4,4-tetramethyl-7-oxa-3,20-diaza-21-oxo-dispiro[5.1.11.2]heneicosan,

3-(N,N-Dioctyl)-carbamoyl-2,2,4,4-tetramethyl-20-octadecyl-7-oxa-3,20-diaza-21-oxo-dispiro[5.1.11.2]heneicosan,

3-Phenylcarbamoyl-2,2,4,4-tetramethyl-20-octadecyl-7-oxa-3,20-diaza-21-oxo-dispiro[5.1.11.2]heneicosan,

8-Cyclohexylcarbamoyl-2,2,7,7,9,9-hexamethyl-3-hexyl-1-oxa-3,8-diaza-4-oxo-spiro[4,5]decan,

Beispiele der Formel Ia mit n = 2 sind:

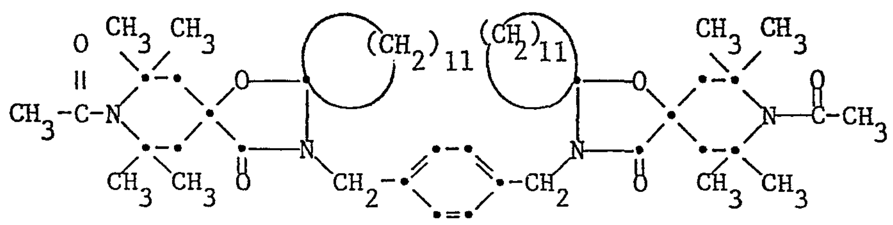

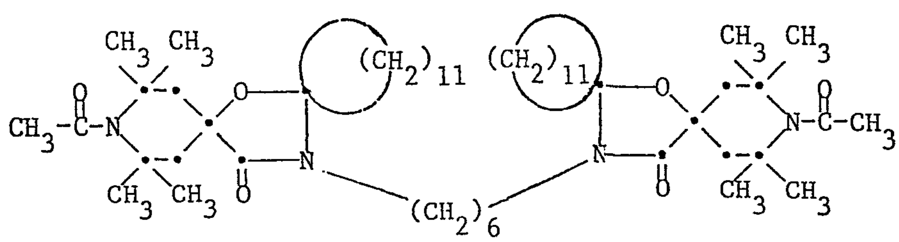

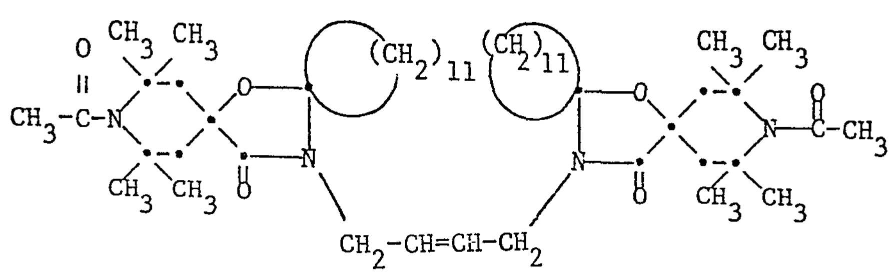

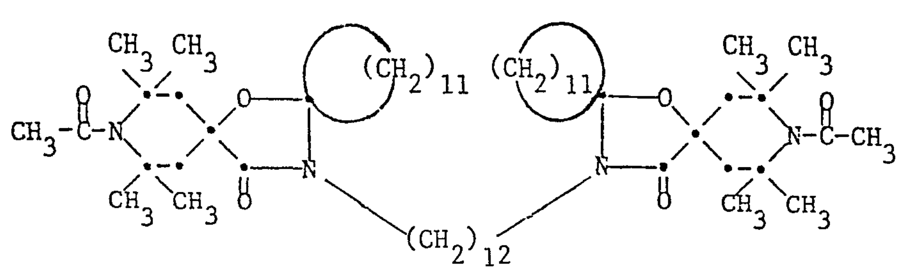

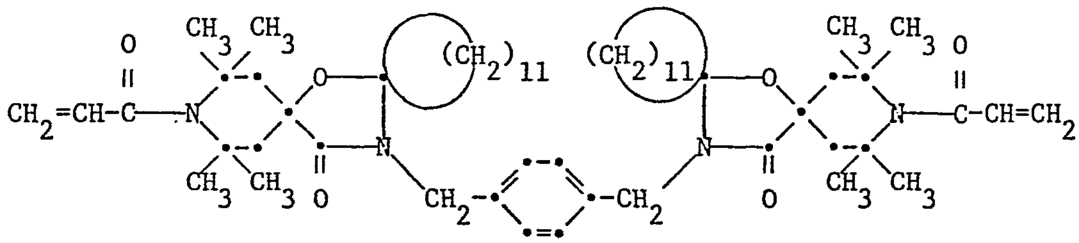

Beispiele für Verbindungen der Formel Ib mit n = 1 sind:

8-Acetyl-2,2,4,7,7,9,9-heptamethyl-1-oxa-4,8-diaza-3-oxo-spiro[4.5]-decan,

8-Acetyl-2,2,7,7,9,9-hexamethyl-1-oxa-4,8-diaza-3-oxo-spiro[4.5]decan,

8-Acetyl-7,7,9,9-tetramethyl-2-cyclohexyl-4-allyl-1-oxa-4,8-diaza-3-oxo-spiro[4.5]decan,

8-Benzylcarbonyl-7,7,9,9-tetramethyl-2,2-dibenzyl-1-oxa-4,8-diaza-3-oxo-spiro[4.5]decan,

8-Decanoyl-2,2,7,7,9,9-hexamethyl-1-oxa-4,8-diaza-3-oxo-spiro[4.5]-decan,

8-Methacryloyl-2,2,4,7,7,9,9-heptamethyl-1-oxa-4,8-diaza-3-oxo-spiro[4.5]decan,

8-Acetyl-2,2,7,7,9,9-hexamethyl-4-benzyl-1-oxa-4,8-diaza-3-oxo-spiro[4.5]decan,

8-Acryloyl-2,2,7,7,9,9-hexamethyl-1-oxa-4,8-diaza-3-oxo-spiro[4.5]-decan,

8-[N,N-Dibutyl]-carbamoyl-2,2,7,7,9,9-hexamethyl-1-oxa-4,8-diaza-3-oxo-spiro[4.5]decan,

4,8-Bisacryloyl-2,2,7,7,9,9-hexamethyl-1-oxa-4,8-diaza-3-oxo-spiro[4.5]decan,

3-Acetyl-2,2,4,4-tetramethyl-7-oxa-3,21-diaza-20-oxo-dispiro-[5.1.11.2]heneicosan,

3,21-Diacetyl-2,2,4,4-tetramethyl-7-oxa-3,21-diaza-20-oxo-dispiro[5.1.11.2]heneicosan,

3-Acryloyl-2,2,4,4-tetramethyl-7-oxa-3,21-diaza-20-oxo-dispiro-[5.1.11.2]heneicosan,

3-(N,N-Diethyl]-carbamoyl-2,2,4,4-tetramethyl-7-oxa-3,21-diaza-20-oxo-dispiro[5.1.11.2]heneicosan.

Beispiele für Verbindungen der Formel Ib mit n = 2 sind

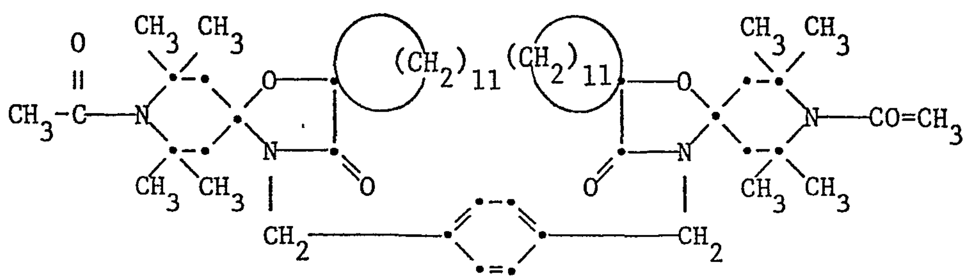

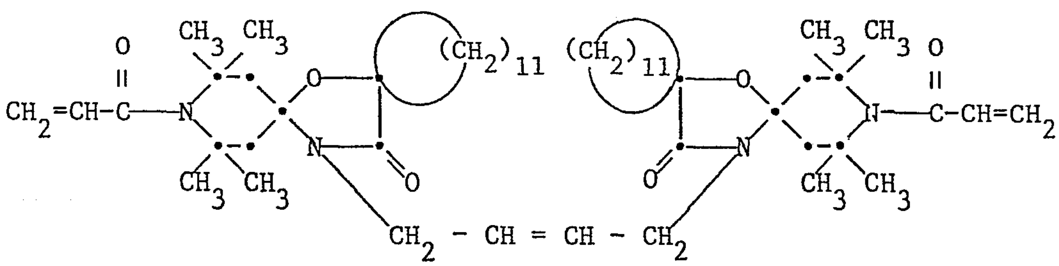

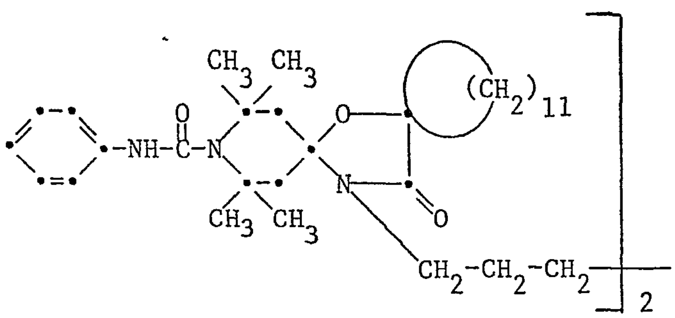

Die Verbindungen der Formel Ia können aus den entsprechenden Grundkörpern der Formel IIa

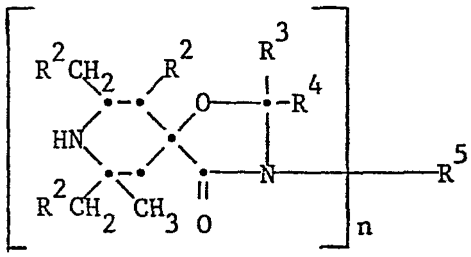

IIa

durch Substitution am Piperidin-Stickstoff hergestellt werden. Hierfür kommen die allgemein zur N-Acylierung von sekundären Aminen bekannten Methoden in Frage. Alkanoyl-, Alkenoyl-, Aroyl-, Arylalkanoyl- und Alkylaroylreste können durch Reaktion mit den entsprechenden Carbonsäurechloriden $R^1Cl$ in Gegenwart eines Protonenakzeptors oder mit den Carbonsäureanhydriden $(R^1)_2O$ eingeführt werden. Ein Carbamoylrest $-CO-N(R^6)(R^7)$ kann durch stufenweise Reaktion von IIa mit a) Phosgen und b) einem Amin $R^6-NH-R^7$ oder durch Reaktion mit einem Carbamoylchlorid $(R^6)(R^7)N-CO-Cl$ eingeführt werden. Carbamoylreste, in denen $R^6$ Wasserstoff ist, erhält man aus IIa durch Umsetzung mit einem Isocyanat $R^7-NCO$.

Geht man dabei von einer Verbindung IIa aus, in der $R^5$ Wasserstoff ist, so kann man bei diesen Acylierungsreaktionen auch gleichzeitig beide Stickstoffatome substituieren und erhält Verbindungen der Formel Ia, worin $R^5$ gleich $R^1$ ist.

Die Einführung eines Cyanomethylrestes als $R^1$ kann durch Reaktion von IIa mit Formaldehyd und Cyanwasserstoff bzw. Formaldehyd und einem Alkalicyanid bewerkstelligt werden.

Die Verbindungen der Formel Ib können in analoger Weise aus den Verbindungen der Formel IIb

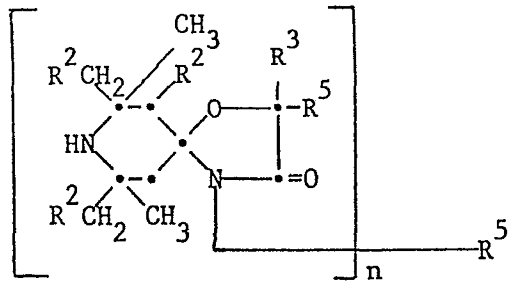

IIb

durch Substitution am Piperidin-Stickstoff hergestellt werden.

Verbindungen der Formel IIa und IIb sind bekannte Verbindungen. Ihre Herstellung ist beispielsweise in der DE-OS 26 06 026, DE-OS 28 34 962 und DE-OS 29 33 732 beschrieben oder kann in dazu analoger Weise bewerkstelligt werden.

Die Verbindungen der Formel Ia und Ib sind als Stabilisatoren für organische Polymere gegen die Einwirkung von Licht, Hitze und Sauerstoff geeignet und zeichnen sich vor allem durch eine hervorragende Lichtschutzwirkung aus. Beispiele für solche Polymere sind die folgenden:

1. Polymere von Mono- und Diolefinen, beispielsweise Polyethylen (das gegebenenfalls vernetzt sein kann), Polypropylen, Polyisobutylen, Polybuten-1-, Polymethylpenten-1, Polyisopren oder Polybutadien sowie Polymerisate von Cycloolefinen, wie z.B. von Cyclopenten oder Norbornen.

2. Mischungen der unter 1) genannten Polymeren, z.B. Mischungen von Polypropylen mit Polyäthylen oder mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (ionomere), sowie Terpolymere von Ethylen mit Propylen und einem Dien, wie Hexadien, Dicyclopentadien oder Ethylidennorbornen.

4. Polystyrol.

5. Copolymere von Styrol oder α-Methylstyrol mit Dienen oder Acrylderivaten, wie z.B. Styrol-Butadien, Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo-und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxyd enthalten.

13. Polyphenylenoxyde und -sulfide.

14. Polyurethane, die sich Polyethern, Polyestern und Polybutadienen mit endständigen Hydroxylgruppen einerseits und aliphatischen oder

aromatischen Polyisocyanaten andererseits ableiten, sowie deren Vorprodukte (Polyisocyanate, Polyole, Präpolymere).

15. Polyamide und Copolyamide, die sich von Diaminen und Dicarbonsäuren und/oder von Aminocarbonsäuren oder den entsprechenden Lactamen ableiten, wie Polyamid, 4, Polyamid 6, Polyamid 6/6, Polyamid 6/10, Polyamid 11, Polyamid 12, Poly-2,4,4-trimethylhexamethylen-terephthalamid, Poly-m-phenylen-isophthalamid, sowie deren Copolymere mit Polyethern, wie z.B. mit Polyethylenglykol, Polypropylenglykol oder Polytetramethylenglykol.

16. Polyharnstoffe, Polyimide und Polyamid-imide.

17. Polyester, die sich von Dicarbonsäuren und Diolen und/oder von Hydroxycarbonsäuren oder den entsprechenden Lactonen ableiten, wie Polyethylenterphthalat, Polybutylenterphthalat, Poly-1,4-dimethylolcyclohexanterephthalat, Poly[2,2-bis(4-hydroxyphenyl)-propan]terephthalat, Polyhydroxybenzoate, sowie Block-Polyether-ester, die sich von Polyethern mit Hydroxyendgruppen, Dialkoholen und Dicarbonsäuren ableiten.

18. Polycarbonate.

19. Polysulfone und Polyethersulfone.

20. Vernetzte Polymere, die sich von Aldehyden einerseits und Phenolen, Harnstoff und Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymeranalog chemisch abgewandelten Derivate, wie Celluloseacetate, -propionate und -butyrate, bzw. die Celluloseether, wie Methylcellulose.

Von besonderer Bedeutung ist die Stabilisierung von Lackharzen wie Alkyd-, Acryl- und Polyesterharzen, insbesondere in ihrer Verwendung für säurekatalysierte Einbrennlacke.

Die erfindungsgemässen Stabilisatoren werden den Polymeren in einer Konzentration von 0,01 bis 5 Gew.-%, berechnet auf das zu stabilisierende Material, zugesetzt. Vorzugsweise werden 0,1 bis 2 Gew.-% der Verbindungen, berechnet auf das zu stabilisierende Material, in dieses eingearbeitet.

Die Einarbeitung kann vor, während oder nach der Polymerisation erfolgen, beispielsweise durch Einmischen der Verbindungen und gegebenenfalls weiterer Additive in die Schmelze nach den in der Technik

üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die Stabilisatoren können auch in Form eines Masterbatches, der diese Verbindungen beispielsweise in einer Konzentration von 2,5 bis 25 Gew.-% enthält, den zu stabilisierenden Polymeren zugesetzt werden.

Ausser den Verbindungen der Formeln Ia und Ib können den Polymeren auch noch andere, bekannte Stabilisatoren zugesetzt werden. Dies können z.B. Antioxydantien, Lichtschutzmittel oder Metalldesaktivatoren sein, oder auch Costabilisatoren wie z.B. solche vom Typ der Phosphorigsäureester. Weiterhin können sonstige in der Kunststofftechnologie übliche Zusätze wie z.B. Flammschutzmittel, Antistatika, Weichmacher, Gleitmittel, Treibmittel, Pigmente, Verstärkungsstoffe oder Füllstoffe zugesetzt werden.

Bei der Mitverwendung bekannter Stabilisatoren können synergistische Effekte auftreten, was besonders bei Mitverwendung von anderen Lichtschutzmitteln oder von organischen Phosphiten häufig der Fall ist. Von besonderer Bedeutung ist die Mitverwendung von Antioxydantien bei der Stabilisierung von Polyolefinen.

Die Erfindung betrifft daher auch die durch Zusatz von 0,1 bis 5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden, z.B. als Folien, Fasern, Bändchen, Profile, insbesondere aber als Bindemittel für Lacke.

Die Herstellung und Verwendung der erfindungsgemässen Verbindungen wird in den folgenden Beispielen näher beschrieben. Teile bedeuten darin Gewichtsteile und % Gewichtsprozente. Die Temperaturen sind in Celsius-Graden angegeben.

Beispiel 1: 51 g (0,14 Mol) 2,2,4,4-Tetramethyl-7-oxa-3,20-diaza-21-oxo-dispiro[5,1,11,2]heneicosan werden in 400 ml Essigsäureanhydrid und 4 ml Dimethylformamid 22 Std. auf 110-115° erhitzt. Hierauf wird das Reaktionsgemisch unter Rühren auf Raumtemperatur gekühlt und filtriert. Das Filtrat wird wie weiter unten angegeben behandelt. Das erhaltene Kristallisat wird aus Dimethylformamid umkristallisiert, mit Wasser und anschliessend mit Aceton gewaschen und im Vakuum getrocknet. Man erhält das 3-Acetyl-2,2,4,4-tetramethyl-7-oxa-3,20-diaza-21-oxo-dispiro[5.1.11.2]heneicosan der Formel

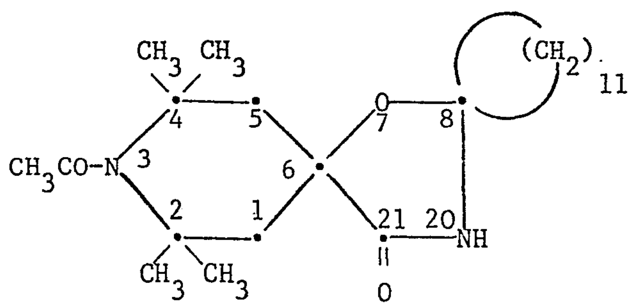

das bei 234-236° schmilzt (Stabilisator Nr. 1).

| | | | | |
|---|---|---|---|---|
| Analyse $C_{24}H_{42}N_2O_3$ | Ber: | C 70,89 | H 10,41 | N 6,89% |
| (406,61) | Gef. | C 70,6 | H 10,5 | N 7,0 % |

Das oben erhaltene Essigsäureanhydrid-Filtrat wird im Vakuum zur Trockene eingedampft. Der erhaltene Rückstand, der nach kurzer Zeit kristallisiert, wird zweimal aus Acetonitril umkristallisiert, wodurch man das reine 3,20-Diacetyl-2,2,4,4-tetramethyl-7-oxa-3,20-diaza-21-oxo-dispiro[5.1.11.2]heneicosan der Formel

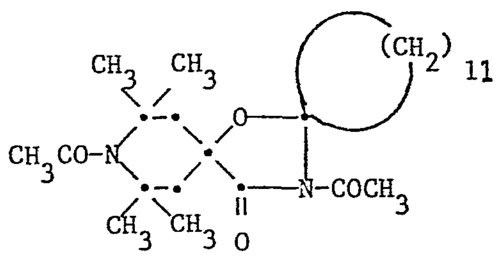

erhält, das bei 183-185° schmilzt.

| | | | | |
|---|---|---|---|---|
| Analyse $C_{26}H_{44}N_2O_4$ | Ber: | C 69,60 | H 9,88 | N 6,24% |
| (448,65) | Gef: | 69,6 | 9,8 | 6,4 % |

Die Mono- und Diacetylverbindung lassen sich auch dünnschichtchromatographisch gut unterscheiden und zeigen im $^1$H-NMR-Spektrum die erwarteten Signale.

Beispiel 2: 27,3 g 2,2,4,4-Tetramethyl-20-benzyl-7-oxa-3,20-diaza-21-oxo-dispiro[5.1.11.2]heneicosan werden in 130 ml Toluol gelöst.Nach der Zugabe von 5,4 g Paraformaldehyd wird der Kolbeninhalt auf 85° erwärmt. Bei dieser Temperatur leitet man während ca. 10 Stunden Blausäure in das Reaktionsgemisch ein (insgesamt 8,2 g), rührt anschliessend noch 12 Stunden bei 85° nach und leitet dann immer bei der gleichen Temperatur während 6 Stunden Stickstoff durch die gelbe Lösung. Das Lösungsmittel wird im Vakuum eingedampft und der Rückstand aus Methyläthylketon unter Zugabe von Aktivkohle umkristallisiert. Man erhält das 3-Cyanomethyl-2,2,4,4-tetramethyl-20-benzyl-7-oxa-3,20-diazo-21-oxo-dispiro[5.1.11.2]heneicosan, das bei 191 - 192° schmilzt (Stabilisator Nr. 2).

| | | | | |
|---|---|---|---|---|
| Analyse $C_{31}H_{47}N_3O_2$ | Ber.: | C 75,41 | H 9,60 | N 8,51 % |
| (493,7) | Gef.: | 75,1 | 9,3 | 8,4 % |

Beispiel 3: 25,0 g (55 mMol) 2,2,4,4-Tetramethyl-20-benzyl-7-oxa-3,20-diaza-21-oxo-dispiro[5.1.11.2]heneicosan, 9,5 ml Triethylamin und 100 mg 2,6-Di-tert.-butyl-4-methylphenol werden in 70 ml Chloroform gelöst und die Lösung auf -12° abgekühlt. Zu dieser Lösung tropft man bei -10° bis -12° in ca. 1 Stunde eine Lösung von 5,23 g (58 mMol) Acrylsäurechlorid in 10 ml Chloroform. Anschliessend wird das Reaktionsgemisch 4 Stunden bei -10° nachgerührt, der Kolbeninhalt mit 250 ml Hexan verdünnt, geklärt und 4 mal mit je 25 ml Wasser extrahiert. Die organische Phase wird über $Na_2SO_4$ getrocknet und dann im Vakuum eingedampft. Durch Kristallisation des Rückstandes aus Acetonitril/Methylethylketon erhält man das 3-Acryloyl-2,2,4,4-tetramethyl-20-benzyl-7-oxa-3,20-diaza-21-oxo-dispiro[5.1.11.2]heneicosan mit einem Schmelzpunkt von 174 - 176° (Stabilisator Nr. 3).

| | | | | | |
|---|---|---|---|---|---|
| Analyse $C_{32}H_{48}N_2O_3$: | Ber.: | C 75,55 | H 9,51 | N 5,51 | % |
| (508,7) | Gef.: | 75,4 | 9,3 | 5,5 | %. |

In analoger Weise wird aus 2,2,4,4-Tetramethyl-20-alkyl-7-oxa-3,20-diaza-21-oxo-dispiro[5.1.11.2]heneicosan und Diethylcarbamoylchlorid das 3-Diethylcarbamoyl-2,2,4,4-tetramethyl-20-alkyl-7-oxa-3,20-diaza-21-oxo-dispiro[5.1.11.2]heneicosan vom Schmelzpunkt 99 - 101° (Stabilisator Nr. 4).

| | | | | | |
|---|---|---|---|---|---|
| Analyse $C_{30}H_{53}N_3O_3$ | Ber.: | C 71,53 | H 10,60 | N 8,34 | % |
| (503,8) | Gef.: | 71,7 | 10,4 | 8,1 | %. |

Beispiel 4: 13,6 g (30 mMol) 2,2,4,4-Tetramethyl-20-benzyl-7-oxa-3,20-diaza-21-oxo-dispiro[5.1.11.2]heneicosan werden mit 0,03 g 1,4-Diazabicyclo[2.2.2]octan (DABCO) in 70 ml Benzol gelöst. Zu dieser Lösung tropft man bei Raumtemperatur in ca. 45 Minuten eine Lösung von 2,97 g (30 mMol) Butylisocyanat in 10 ml Benzol und lässt dann während 24 Stunden bei Raumtemperatur ausreagieren. Die Reaktionslösung wird zur Hälfte eingedampft und mit 200 ml Diethylether verdünnt. Der entstandene farblose Niederschlag wird abgenutscht mit Ethylether gewaschen und getrocknet. Das erhaltene 3-Butyl-carbamoyl-2,2,4,4-tetramethyl-20-benzyl-7-oxa-3,20-diaza-21-oxo-dispiro[5.1.11.2]heneicosan schmilzt bei 148 - 150° (Stabilisator Nr. 5)

| | | | | | |
|---|---|---|---|---|---|
| Analyse $C_{34}H_{55}N_3O_3$ | Ber.: | C 73,74 | H 10,01 | N 7,59 | % |
| (553,8) | Gef.: | 73,3 | 10,0 | 7,9 | % |

Bei gleicher Arbeitsweise werden aus den entsprechenden Ausgangsprodukten erhalten:

3-Cyclohexylcarbamoyl-2,2,4,4-tetramethyl-20-allyl-7-oxa-3,20-diaza-21-oxo-dispiro[5.1.11.2]heneicosan mit einem Schmelzpunkt von 140 - 142° (Stabilisator Nr. 6),

3-Phenylcarbamoyl-2,2,4,4-tetramethyl-20-allyl-7-oxa-3,20-diaza-21-oxo-dispiro[5.1.11.2]heneicosan mit einem Schmelzpunkt von 124 - 127° (Stabilisatoren Nr. 7).

Beispiel 5: 19,6 g 2,2,4,4-Tetramethyl-20-allyl-7-oxa-3,20-diaza-21-oxo-dispiro[5.1.11.2]heneicosan und 80 ml Essigsäureanhydrid werden während 24 Stunden bei 90 - 95° gerührt. Das überschüssige Anhydrid und die entstandene Essigsäure werden im Vakuum möglichst vollständig abdestilliert. Der kristalline Rückstand wird aus Acetonitril umkristallisiert. Das erhaltene 3-Acetyl-2,2,4,4-tetramethyl-20-alkyl-7-oxa-3,20-diaza-21-oxo-dispiro[5.1.11.2]heneicosan schmilzt bei 113 - 114° (Stabilisator Nr. 8).

| | | | | | |
|---|---|---|---|---|---|
| Analyse $C_{27}H_{46}N_2O_3$ | Ber.: | C 72,6 | H 10,38 | N 6,27 | % |
| (446,8) | Gef.: | 72,5 | 10,2 | 6,4 | % |

Bei gleicher Arbeitsweise werden aus den entsprechenden Ausgangsprodukten erhalten:

3-Acetyl-2,2,4,4-tetramethyl-20-benzyl-7-oxa-3,20-diaza-21-oxo-dispiro-[5.1.11.2]-heneicosan mit einem Schmelzpunkt von 128 - 129° (Stabilisator Nr. 9),

3-Acetyl-2,2,4,4-tetramethyl-20-butylcarbamoyl-7-oxa-3,20-diaza-21-oxo-dispiro[5.1.11.2]heneicosan mit einem Schmelzpunkt von 176 - 178° (Stabilisator Nr. 10),

3-Acetyl-2,2,4,4-tetramethyl-20-hexyl-7-oxa-3,20-diaza-21-oxo-dispiro-[5.1.11.2]heneicosan mit einem Schmelzpunkt von 89 - 91° (Stabilisator Nr. 11),

1,4-Bis(3-acetyl-2,2,4,4-tetramethyl-7-oxa-3,20-diaza-21-oxo-dispiro-[5.1.11.2]heneicosyl-20)-buten-2 mit einem Schmelzpunkt von 156 - 158° (Stabilisator Nr. 12).

Beispiel 6: Zu einer Lösung von 20,2 g 2,2,4,4-Tetramethyl-20-alkyl-7-oxa-3,20-diaza-21-oxo-dispiro[5.1.11.2]heneicosan in 120 ml Ethylacetat wird unter Rühren bei 0° innerhalb von 1 Stunde die Lösung von 2,48 g Phosgen in 10 ml Ethylacetat getropft. Nach weiteren 16 Stunden Rühren bei Raumtemperatur wird eine Lösung von 1,5 g Ethanol und 2,5 g Triethylamin in 10 ml Ethylacetat zugetropft. Nach 8 Stunden Rühren wird das Reaktionsgemisch filtriert und das Filtrat zweimal mit Wasser, dreimal mit eiskalter n-HCl und noch einmal mit Wasser gewaschen, über $Na_2SO_4$ getrocknet und im Vakuum eingedampft.
Das hinterbleibende Rohprodukt wird durch Chromatographie an einer Kieselgel-Säure gereinigt (Eluierungsmittel: Diethylether-Hexan 3:2) und aus Pentan umkristallisiert. Man erhält das reine 3-Ethoxycarbonyl-2,2,4,4-tetramethyl-20-alkyl-7-oxa-3,20-diaza-21-oxo-dispiro[5.1.11.2]-heneicosan vom Schmelzpunkt 81 - 82° (Stabilisator Nr. 13)

| | | | | | |
|---|---|---|---|---|---|
| Analyse | $C_{28}H_{48}N_2O_4$ | Ber.: | C 70,55 | H 10,15 | N 5,88 % |
| | (476,7) | Gef.: | 70,8 | 10,0 | 6,0 %. |

Das $^{1}H$-NMR-Spektrum steht mit der angegebenen Struktur im Einklang.

Beispiel 7: Stabilisierung von Polypropylen gegen Licht.

100 Teile Polypropylenpulver (Moplen, Fibre grade, der Firme Montedison) werden mit 0,2 Teilen β-(3,5-Di-tert.-butyl-4-hydroxyphenyl)-propionsäure-octadecylester, 0,1 Teilen Calciumstearat und 0,25 Teilen eines Stabilisators der folgenden Tabelle 1 im Brabender-Plastographen bei 200° während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer hydraulischen Laborpresse während 6 Minuten bei 260° zu einer 0,1 mm dicken Folie gepresst, die unverzüglich in kaltem Wasser abgeschreckt wird. Aus dieser werden nun Abschnitte ausgestanzt und im Xenotest 1200 belichtet. In regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µm während der Belichtung ist ein Mass für den photooxidativen Abbau des Polymeren (s.L. Balaban et al., J. Polymer Sci, Part C; 22 (1969), 1059 - 1071) und ist erfahrungsgemäss mit einem Abfall der mechanischen Eigenschaften des Polymeren verbunden. Als Mass der Schutzwirkung gilt die Zeit bis Erreichen einer Carbonylextinktion von ca. 0,3, bei welcher die Vergleichsfolie brüchig ist.

Tabelle 1

| Stabilisator Nr. | Belichtungszeit in Stunden |
| --- | --- |
| 2 | > 2100 |
| 3 | > 2200 |
| 4 | > 2200 |
| 5 | > 1600 |
| 6 | > 2200 |
| 7 | > 1700 |
| 8 | > 2200 |
| 9 | > 2200 |
| 10 | > 1700 |
| ohne | 880 |

Beispiel 8: Stabilisierung eines Zweischicht-Metallic-Einbrennlackes

Basislack-Rezeptur

27 Teile Polyesterharz (L 1850, Dynamit Nobel AG)
3 Teile Melaminharz (Maprenal® RT, Hoechst AG)
2 Teile Celluloseacetobutyrat (CAB 531, Eastman Chem. Corp.)
8 Teile Aluminiumbronze (Alcoa® 726, Aluminium Corp. of USA)
10 Teile Toluol
7 Teile Xylol
3 Teile Butanol
25 Teile Butylacetat
15 Teile aromatisches Lösungsmittelgemisch (Solvesso® 150, Esso AG)

Decklack-Rezeptur

58,3 Teile Acrylharz (Viacryl® VC 373, Vianova AG)
27,3 Teile Melaminharz (Maprenal® MF 590, Hoechst AG)
1,0 Teile 1 %ige Lösung eines Siliconöles in Xylol
4,0 Teile aromatisches Lösungsmittelgemisch (Solvesso®, Esso AG)

5,4 Teile Xylol

4,0 Teile Ethylglycolacetat

Zur Lichtstabilisierung werden dem Decklack 0,6 Teile eines erfindungsgemässen Stabilisators zugemischt, was einer Konzentration von 1 Gew.-% bezogen auf den Feststoffgehalt des Klarlackes entspricht.

Der pigmentierte Basislack wird in einer Schichtdicke von 15 µm auf Aluminiumbleche, die mit einem Polyester-Epoxidharz-Lack grundiert sind, gespritzt. Nach 10 Minuten wird der Decklack in einer Schichtdicke von 30 µm darübergespritzt. Nach einer Ablüftzeit von 15 Minuten werden die Proben 30 Minuten bei 120° eingebrannt.

Die lackierten Bleche werden eine Woche im Normklima (23°C/5 % RLF) gelagert und anschliessend in einem QUV-Gerät einer Bewitterung gemäss ASTM G 53 - 77 unterworfen. Hierbei werden die Proben jeweils 4 Stunden bei 50° befeuchtet und 4 Stunden in feuchter Atmosphäre bei 60° durch UV-Licht bestrahlt.

Die Proben werden in regelmässigen Abständen kontrolliert und ihr Aussehen nach der TNO-Rissbildskala beurteilt. Diese Skala hat 10 Stufen der Intensität (1-10) und klassifiziert verschiedene Typen von Rissbildern (A-P) und Risstiefe (a-f). Die Bewitterungszeit im QUV-Gerät bis zu einer Rissbildung von 6 - 8b gemäss TNO-Skala wird in der folgenden Tabelle 2 als Riss-Standzeit angegeben.

Tabelle 2

| Stabilisator Nr. | Riss-Standzeit in Stunden |
| --- | --- |
| 1a | > 1500 |
| 2 | > 1500 |
| 7 | > 1500 |
| 9 | > 1500 |
| ohne | 1100 |

0079850

Patentansprüche

1. Verbindungen der Formel Ia oder Ib,

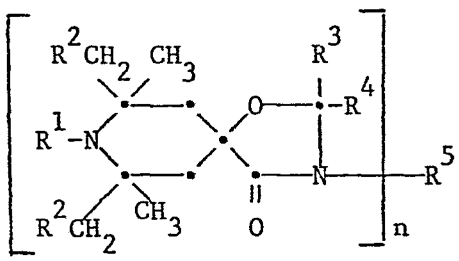

(Ia)

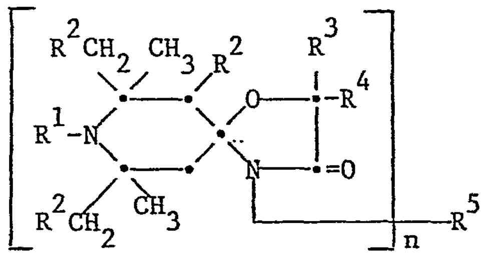

(Ib)

worin n 1 oder 2 ist,

$R^1$ $C_2$-$C_{30}$-Alkanoyl, $C_3$-$C_{20}$-Alkenoyl, $-CH_2CN$, $-CO-N(R^6)(R^7)$, $C_7$-$C_{11}$-Aroyl, $C_8$-$C_{14}$ Aryl-alkanoyl oder $C_8$-$C_{20}$ Alkylaroyl bedeutet,

$R^2$ Wasserstoff oder $C_1$-$C_4$-Alkyl ist,

$R^3$ und $R^4$ unabhängig voneinander Wasserstoff, $C_1$-$C_{30}$-Alkyl, unsubstituiertes oder durch Cl oder Alkyl substituiertes $C_6$-$C_{10}$-Aryl, unsubstituiertes oder durch Alkyl substituiertes $C_7$-$C_{11}$-Aralkyl bedeuten oder $R^3$ und $R^4$ zusammen mit dem C-Atom, an das sie gebunden sind, einen Cycloalkan- oder Alkylcycloalkanring mit 5-18 C-Atomen oder eine Gruppe der Formel

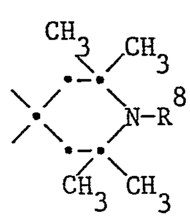

bilden,

$R^5$ wenn n = 1, $C_1$-$C_{30}$-Alkyl, $C_3$-$C_{20}$-Alkenyl, unsubstituiertes oder durch Alkyl substituiertes $C_7$-$C_{11}$-Aralkyl oder $C_5$-$C_{12}$-Cycloalkyl, $C_2$-$C_{18}$-Alkanoyl, $C_3$-$C_{18}$-Alkenoyl,

$C_7$-$C_{11}$-Aroyl, $C_8$-$C_{14}$-Aryl-alkanoyl oder $C_8$-$C_{20}$-Alkyl-aroyl bedeutet,

$R^5$ wenn n = 2, $C_2$-$C_{30}$-Alkylen, $C_2$-$C_{30}$-Alkenylen oder $C_8$-$C_{18}$-Arylen-dialkylen bedeutet,

$R^6$ Wasserstoff, $C_1$-$C_{18}$-Alkyl, $C_5$-$C_8$-Cycloalkyl, $C_7$-$C_{15}$-Aralkyl oder $C_6$-$C_{10}$-Aryl bedeutet,

$R^7$ $C_1$-$C_{18}$-Alkyl, $C_5$-$C_8$-Cycloalkyl, $C_7$-$C_{15}$-Aralkyl oder $C_6$-$C_{10}$-Aryl bedeutet, oder

$R^6$ und $R^7$ zusammen mit dem N-Atom einen 5-7-gliedrigen, gegebenenfalls durch weitere Heteroatome unterbrochenen Ring bilden und

$R^8$ Wasserstoff, $C_1$-$C_{12}$-Alkyl, $C_3$-$C_5$-Alkenyl, $C_3$-$C_5$-Alkinyl, unsubstituiertes oder durch Alkyl substituiertes $C_7$-$C_{11}$-Aralkyl, $C_1$-$C_{18}$-Alkanoyl, $C_3$-$C_{18}$-Alkenoyl, $-CH_2CN$ oder $-CO-N(R^6)(R^7)$ bedeutet.

2. Verbindungen gemäss Anspruch 1 der Formel Ia oder Ib, worin

$R^1$ $C_2$-$C_{12}$-Alkanoyl, $C_3$-$C_5$-Alkenoyl, Phenylacetyl, $-CH_2CN$ oder $-CO-N(R^6)(R^7)$ bedeutet,

$R^2$ Wasserstoff ist,

$R^3$ und $R^4$ unabhängig voneinander Wasserstoff, $C_1$-$C_{12}$-Alkyl, $C_7$-$C_{12}$-Aralkyl oder durch $C_1$-$C_4$-Alkyl substituiertes Phenyl bedeuten oder $R^3$ und $R^4$ zusammen mit dem C-Atom, an das sie gebunden sind, einen Cycloalkan- oder Alkylcycloalkanring mit 5-12 C-Atomen oder eine Gruppe der Formel

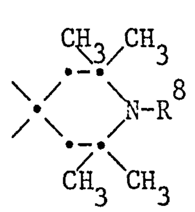

bilden,

$R^5$ wenn n = 1, $C_1$-$C_{18}$-Alkyl, $C_3$-$C_6$-Alkenyl, $C_5$-$C_6$-Cycloalkyl, $C_7$-$C_{12}$-Aralkyl, $C_2$-$C_{12}$-Alkanoyl oder $C_3$-$C_{12}$-Alkenoyl, und wenn n = 2, $C_2$-$C_{18}$-Alkylen, $C_4$-$C_{12}$-Alkenylen oder Xylylen bedeutet,

$R^6$ Wasserstoff, $C_1$-$C_{12}$-Alkyl, $C_5$-$C_6$-Cycloalkyl, $C_7$-$C_{12}$-Aralkyl oder Phenyl bedeutet,

$R^7$ $C_1$-$C_{12}$-Alkyl, $C_5$-$C_6$-Cycloalkyl, $C_7$-$C_{12}$-Aralkyl oder $C_6$-$C_{10}$-Aryl bedeutet oder $R^6$ und $R^7$ zusammen mit dem N-Atom einen 5-7-gliedrigen Ring bilden und

$R^8$ Wasserstoff, $C_1$-$C_6$-Alkyl, $C_3$-$C_5$-Alkenyl, Benzyl, $C_2$-$C_{12}$-Alkanoyl, $C_3$-$C_{12}$-Alkenoyl, Propargyl oder Cyanomethyl bedeutet.

3. Verbindungen gemäss Anspruch 1 der Formel Ia, worin n = 1 ist, $R^1$ $C_2$-$C_{12}$-Alkanoyl, $C_3$-$C_5$-Alkenoyl, $-CO-N(R^6)(R^7)$ oder $-CH_2CN$ bedeutet, $R^2$ Wasserstoff ist, $R^3$ und $R^4$ unabhängig voneinander H, $C_1$-$C_4$-Alkyl, Benzyl oder Phenyl bedeuten oder $R^3$ und $R^4$ zusammen mit dem C-Atom, an das sie gebunden sind, einen $C_5$-$C_{12}$-Cycloalkanring bilden, $R^5$ $C_1$-$C_6$-Alkyl, $C_3$-$C_5$-Alkenyl, Benzyl, $C_2$-$C_{12}$-Alkanoyl oder $C_3$-$C_5$-Alkenoyl bedeutet, $R^6$ H, $C_1$-$C_6$-Alkyl, Cyclohexyl, Benzyl oder Phenyl bedeutet und $R^7$ $C_1$-$C_6$-Alkyl, Cyclohexyl, Benzyl oder Phenyl bedeutet oder $R^6$ und $R^7$ zusammen mit dem N-Atom einen 5-7-gliedrigen Ring bilden.

4. Verwendung der Verbindungen des Anspruches 1 als Stabilisatoren für organische Polymere gegen Schädigung durch Licht, Hitze und Sauerstoff.

5. Gegen Licht-, Hitze- und Sauerstoff-Einwirkung stabilisierte organische Polymere, welche als Stabilisator 0,01 bis 5 Gew.-%, berechnet auf das zu stabilisierende Polymere, einer Verbindung des Anspruchs 1 enthalten.

Europäisches Patentamt

**EUROPÄISCHER RECHERCHENBERICHT**

0079850
Nummer der Anmeldung

EP 82 81 0419

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 227 689 (SANKYO und CO., LTD.) * Ansprüche 1,2,23; Seite 3, Formel I; Seite 5, Zeilen 11,12,18,29-31; Seite 15, Zeilen 7-10 * | 1-5 | C 07 D 498/10<br>C 07 D 498/20<br>C 08 K 5/35 //<br>(C 07 D 498/10<br>C 07 D 263/00<br>C 07 D 221/00 )<br>(C 07 D 498/20<br>C 07 D 263/00<br>C 07 D 221/00<br>C 07 D 221/00 ) |
| Y | EP-A-0 025 867 (HOECHST AG) * Ansprüche 1,4,10 * | 1-5 | |
| Y | DE-A-2 063 573 (YOSHITOMI PHARMACEUTICAL INDUSTRIES LTD.) * Ansprüche 1,6; Seite 15, Zeilen 8-11; Seite 16, Zeilen 12,13 * | 1-3 | |
| | | | RECHERCHIERTE SACHGEBIETE (Int. Cl. 3) |
| | | | C 07 D 498/10<br>C 07 D 498/20<br>C 08 K 5/35 |

Der vorliegende Recherchenbericht wurde für alle Patentansprüche erstellt.

| Recherchenort | Abschlußdatum der Recherche | Prüfer |
|---|---|---|
| BERLIN | 11-01-1983 | HASS C V F |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503. 03 82